## Europäisches Patentamt
## European Patent Office
## Office européen des brevets

(11) Veröffentlichungsnummer: **0 224 872**
**B1**

(12) **EUROPÄISCHE PATENTSCHRIFT**

(45) Veröffentlichungstag der Patentschrift:
10.08.88

(21) Anmeldenummer: **86116461.4**

(22) Anmeldetag: **27.11.86**

(51) Int. Cl.⁴: **C 07 C 29/14,** C 07 C 31/125

(54) **Verfahren zur Herstellung von C3-C25-Alkanolen.**

(30) Priorität: **03.12.85  DE 3542595**

(43) Veröffentlichungstag der Anmeldung:
**10.06.87 Patentblatt 87/24**

(45) Bekanntmachung des Hinweises auf die Patenterteilung:
**10.08.88 Patentblatt 88/32**

(84) Benannte Vertragsstaaten:
**AT BE DE ES FR GB IT NL**

(56) Entgegenhaltungen:
**AT-A-13 874**
**DD-A-106 347**
**DD-A-150 453**
**DE-A-2 321 101**
**DE-B-1 115 232**
**DE-B-1 152 393**
**DE-B-1 161 250**
**DE-B-1 231 227**
**DE-B-1 269 605**
**DE-B-2 257 673**

(73) Patentinhaber: **BASF Aktiengesellschaft, Carl- Bosch- Strasse 38, D-6700 Ludwigshafen (DE)**

(72) Erfinder: **Grenacher, Armin Volker, Dr., An der Steinernen Brücke 10, D-6704 Mutterstadt (DE)**
Erfinder: **Strohmeyer, Max, Dr., Woogstrasse 41, D-6703 Limburgerhof (DE)**
Erfinder: **Hoffmann, Herwig, Dr., Knietschstrasse 21, D-6710 Frankenthal (DE)**
Erfinder: **Elliehausen, Heinrich, Dr., Westhues 32, D-2280 Tinnum/Post Westerland (DE)**

EP 0 224 872 B1

LIBER, STOCKHOLM 1988

# 0 224 872

**Beschreibung**

Die vorliegende Erfindung betrifft ein verbessertes Verfahren zur Herstellung von $C_3$-$C_{25}$-Alkanolen durch katalytische Hydrierung der entsprechenden Rohalkanale, wie sie bei der mit Cobalt katalysierten Hydroformylierung von $C_2$-$C_{24}$-Olefinen anfallen.

Es ist allgemein bekannt, daß man Alkanale mit Wasserstoff und mittels Nickel enthaltender Trägerkatalysatoren zu den entsprechenden Alkanolen hydrieren kann. Handelt es sich um reine Aldehyde, bietet diese Hydrierung keine Probleme, jedoch ist sie im Falle von Rohalkanalen, wie sie bei der cobaltkatalysierten Hydroformylierung der entsprechenden Olefine anfallen, mit erheblichen technischen Schwierigkeiten verbunden, da die Rohalkanale noch diverse Begleitstoffe enthalten, die ebenfalls hydriert werden müssen, sei es, daß sie wie Formiate und Acetale ebenfalls in das gewünschte Alkanol überführt werden müssen,

$$
\left.
\begin{array}{l}
R - CHO \\
R - O - CO - H \\
R - CH(OR)_2
\end{array}
\right\} \xrightarrow{\quad H_2 \quad} R - CH_2OH
$$

$$R = \text{Alkyl}$$

damit kein Alkanol verloren geht, oder sei es, daß die Begleitstoffe, würden sie nicht oder nicht vollständig hydriert werden, erhebliche Qualitatsminderungen der Alkanole bedingen würden.

Da nun die Hydrierung der Begleitstoffe unter sonst gleichen Bedingungen langsamer verläuft als die Hydrierung der Alkanale und da sich hieraus für einen vollständigen Umsatz unwirtschaftlich geringe Raum-Zeit-Ausbeuten ergeben werden, müßte man zur Behebung dieses Nachteils die Reaktionsbedingungen verschärfen, indem man bei höherer Temperatur, höherem Druck, mit einem aktiveren Katalysator und/oder einer größeren Katalysatormenge arbeitet. Dies gäbe allerdings zu neuen Störungen Anlaß, denn erstens besteht die Gefahr, daß die (exotherme) Hydrierung außer Kontrolle gerät und zweitens wird die unerwünschte Fischer-Tropsch-Reaktion, nämlich die Bildung von Paraffinen aus dem von der Formiathydrierung stammenden Kohlenmonoxid begünstigt, wobei die niederen Paraffine die Alkanolqualität beeinträchtigen und die höheren am Kontakt verbleiben und diesen frühzeitig desaktivieren.

Es ist weiterhin allgemein bekannt, für die Hydrierung von Alkanalen Cobaltkatalysatoren zu verwenden. Diese bieten im allgemeinen den Vorteil, daß im Falle der Rohalkanale auch die Begleitstoffe mithydriert werden, ohne daß es zu den obenerwähnten Komplikationen kommt, jedoch sind Cobaltkatalysatoren erheblich teurer als Nickelkatalysatoren. Außerdem weisen sie eine geringere Lebensdauer auf, die umso kürzer wird, je höher die Betriebstemperaturen werden, jedoch sind gerade höhere Temperaturen für einen vollständigen Umsatz erforderlich, will man nicht zu lange Reaktionszeiten in Kauf nehmen.

Der Erfindung lag daher die Aufgabe zugrunde, die geschilderten Nachteile bei der Hydrierung von Rohalkanalen zu vermeiden und das Hydrierverfahren insgesamt wirtschaftlicher zu gestalten als bisher.

Demgemäß wurde ein verbessertes Verfahren zur Herstellung von $C_3$-$C_{25}$-Alkanolen durch katalytische Hydrierung der entsprechenden Rohalkanale, wie sie bei der mit Cobalt katalysierten Hydroformylierung von $C_2$-$C_{24}$-Olefinen anfallen, gefunden, welches dadurch gekennzeichnet ist, daß man

a) die Hydrierung in einer ersten Stufe bis zu einer Wasserstoffaufnahme von 80 - 95 % bei 150 - 230°C und 10 - 350 bar mit einem $SiO_2$-Trägerkatalysator vornimmt, der 5 - 15 Gew.-% Nickel sowie 2 - 20 Gew.-% Molybdän in Form von Molybdänoxid, jeweils bezogen auf seine Gesamtmenge, enthält, und daß man

b) die Hydrierung in einer zweiten Stufe bei 150 - 230°C und 10 - 350 bar mit Hilfe eines (obalt-Katalysators zu Ende führt, dessen aktive Masse aus

    55 - 65 Gew.-% Cobalt
    15 - 20 Gew.-% Kupfer
    4 - 10 Gew.-% Mangan
    2 - 5 Gew.-% Molybdän in Form von Molybdänoxid und
    0 - 10 Gew.-% sonstigen aktivierenden Zusätzen besteht.

Zahlreiche der definitionsgemäßen Nickel/Molybdän-Katalysatoren sind an sich bekannt. Man stellt sie zweckmäßigerweise durch Tränken von Kieselsäure-Trägermaterialien mit einer wäßrigen Lösung eines Nickel- und eines Molybdänsalzes. Formgebung der Masse und anschließendes Trocknen und gegebenenfalls Tempern her. Als Nickelsalze eignen sich hierbei z.B. das Nitrat, das Chlorid, das Formiat und das Acetat, wogegen das Sulfat weniger in Betracht kommt, weil dieses unter den Hydrierbedingungen zum Sulfid reduziert wird, welches den Kontakt desaktivieren kann. Als Molybdänsalz kommt in erster Linie Ammoniummolybdat in Betracht. Neben dem Nickel und dem Molybdän können diese Katalysatoren noch aktivierende Komponenten wie Kupfer und/oder Magnesium enthalten, wobei man diese Metalle auf analoge Weise wie das Nickel und das Molybdän auf das Trägermaterial aufbringt. Die Mengenverhältnisse der genannten Komponenten richten sich dabei nach den entsprechenden Anteilen im fertigen reduzierten Katalysator.

2

Die fertigen Katalysatoren werden sodann zweckmäßigerweise unter Wasserstoffatmosphäre auf etwa 300 - 500°C erhitzt, wobei das Nickel sowie weitere reduzierbare Komponenten in die aktive metallische Form überführt werden. Diese Vorbehandlung ist jedoch nicht unbedingt erforderlich, da sich die aktive Form der Katylysatoren auch unter den Bedingungen der Aldehyd-Hydrierung bildet.

Gut geeignete Katalysatoren dieser Art sowie deren Herstellung sind in der DE-PS-2 257 673 beschrieben. Sie bestehen beispielsweise aus 5 -15 Gew.-% Ni, 5 - 20 Gew.-% Mo in Form von $MoO_3$ und dem Rest eines hitzebehandelten $SiO_2$-Trägermaterials.

Die definitionsgemäßen Cobaltkatalysatoren sowie deren Herstellung sind ebenfalls an sich bekannt, und zwar aus der DE-OS-2 321 101, auf welche hinsichtlich der präparativen Einzelheiten verwiesen sei.

In beiden Stufen des erfindungsgemäßen Verfahrens ordnet man die Katalysatoren zweckmäßigerweise als feste Kontakte in druckfesten Reaktoren oder in einer Anzahl mehrerer Rohre (Rohrbündelreaktor) an. Die erforderlichen Mengen ergeben sich aus einer Belastbarkeit von etwa 0,5 - 1,5 kg Rohalkanal pro l Katalysatorschüttung pro Stunde im Falle der Nickel/Molybdän-Katalysatoren und etwa 1,0 - 3,0 kg Rohalkanal pro l Katalysatorschüttung pro Stunde im Falle der Cobaltkatalysatoren.

In der ersten Hydrierstufe arbeitet man vorzugsweise bei 160 - 220°C und einem $H_2$-Druck von 20 - 280 bar, und für die zweite Stufe empfehlen sich besonders Temperaturen zwischen 155 und 210°C sowie ein $H_2$-Druck von 20 - 280 bar.

Unter diesen Bedingungen nimmt die Hydrierung in der ersten Stufe bei einem 80 - 95 %-igen Umsatz etwa 0,5 - 1,5 Stunden in Anspruch, und für den Restumsatz in der zweiten Stufe sind etwa 20 - 40 Minuten erforderlich.

Aus diesen Werten ergeben sich Raum-Zeit-Ausbeuten von etwa 0,4 - 110 kg $l^{-1}h^{-1}$. Derart günstige Werte lassen sich zwar auch bei alleiniger Verwendung der definitionsgemäßen Cobaltkatalysatoren erzielen, dies aber nur bei deutlich geringerer Gesamtwirtschaftlichkeit, denn erstens sind die Cobaltkatalysatoren erheblich teurer und zweitens weisen sie auch eine wesentlich geringere Lebensdauer auf, jeweils im Vergleich zu den Nickel/Molybdän-Katalysatoren.

Das erfindungsgemäße Verfahren eignet sich für die Hydrierung jeglicher Rohalkanole, welche aus der cobaltkatalysierten Hydroformylierung von $C_2$-$C_{24}$-Olefinen stammen, d.h. welche neben den n- und iso-Alkanolen noch Acetale und Formiate als hauptsächliche Begleitstoffe enthalten. Besondere Bedeutung hat das Verfahren im Rahmen der Herstellung von höheren primären Alkanolen aus α-Olefinen wie $C_9$-$C_{11}$ und $C_{13}$-$C_{15}$-Alkanolen, da diese Alkanole für die Herstellung von hochwertigen Weichmacherestern dienen, an welche hinsichtlich Farbe, Geruch und Anwendungseigenschaften extreme Qualitätsanforderungen gestellt werden.

## Beispiel

3,2 kg eines Roh-$C_9$-$C_{11}$-Alkanals, welches durch cobaltkatalysierte Hydroformylierung eines $C_8$-$C_{10}$-Olefingemisches hergestellt worden war und als Begleitstoffe u.a. 6 Gew.-% $C_9$-$C_{11}$-Alkylformiat und 7 Gew.-% Acetale enthielt, wurden bei 170°C und 250 bar $H_2$-Druck über 4,3 l eines Nickel/Molybdän-Katalysators geleitet, wobei die mittlere Kontaktzeit etwa 1 h betrug. Diese Hydrierung wurde bis zu einem Umsatz von 95 % geführt.

In einer zweiten Hydrierstufe wurde der Umsatz vervollständigt und zwar bei 170°C und 245 bar an 2,2 l eines Cobaltkatalysators bei einer mittleren Kontaktzeit von 0,5 Stunden.

Die Ausbeute an den entsprechenden Alkanolen, bezogen auf die aus der genannten Rohware theoretisch herstellbare Menge, betrug 99,6 %. Dies entsprach einer Raum-Zeit-Ausbeute von rd. 0,5 kg $l^{-1}h^{-1}$.

Der Nickel/Molybdän-Katalysator, der nach der Vorschrift von Beispiel 1 der DE-PS-2 257 673 hergestellt worden war, bestand in reduzierter Form aus 81,7 Gew.-% $SiO_2$, 8,3 Gew.-% Ni und 10,0 Gew.-% Mo (als $MoO_3$). Er hatte die Form von Strängen von 6 mm Durchmesser und 15 - 30 mm Länge.

Der Cobaltkatalysator wurde nach der Vorschrift von Beispiel 1 der DE-OS-2 321 101 hergestellt. In reduzierter Form setzte er sich aus 59,1 Gew.-% Co, 17,2 Gew.-% Cu, 5,8 Gew.-% Mn (als Oxid), 2,7 Gew.-% Mo (als Oxid), 3,2 Gew.-% Phosphorsäure und den Rest aus oxidischem Sauerstoff zusammen. Die strangförmigen Teilchen hatten einen Durchmesser von 4 mm und eine Länge von 7 - 15 mm.

Bei Verwendung des Nickelkatalysators allein konnte nur eine Raum-Zeit-Ausbeute von 0,37 kg $l^{-1}h^{-1}$ erzielt werden.

## Patentanspruch

Verfahren zur Herstellung von $C_3$-$C_{25}$-Alkanolen durch katalytische Hydrierung der entsprechenden Rohalkanale, wie sie bei der mit Cobalt katalysierten Hydroformylierung von $C_2$-$C_{24}$-Olefinen anfallen, dadurch gekennzeichnet, daß man

a) die Hydrierung in einer ersten Stufe bis zu einer Wasserstoffaufnahme von 80 - 95 % bei 150 - 230°C und 10 - 350 bar mit einem $SiO_2$-Trägerkatalysator vornimmt, der 5 - 15 Gew.-% Nickel sowie 2 - 20 Gew.-% Molybdän in Form von Molybdänoxid, jeweils bezogen auf seine Gesamtmenge, enthät, und daß man

**0 224 872**

b) die Hydrierung in einer zweiten Stufe bei 150 - 230° C und 10 - 350 bar mit Hilfe eines Cobalt-Katalysators zu Ende führt, dessen aktive Masse aus

55 - 65 Gew.-% Cobalt
15 - 20 Gew.-% Kupfer
4 - 10 Gew.-% Mangan
2 - 5 Gew.-% Molybdan in Form von Molybdänoxid und
0 - 10 Gew.-% sonstigen aktivierenden Zusätzen besteht.

**Claim**

A process for the preparation of a $C_3$-$C_{25}$-alkanol by catalytic hydrogenation of the corresponding crude alkanal, as obtained in the cobalt-catalyzed hydroformylation of $C_2$-$C_{24}$-olefins, wherein
a) the hydrogenation is carried out in a first stage to a hydrogen absorption of 80 - 95 % at 150 - 230° C and under 10 - 350 bar using an $SiO_2$ supported catalyst which contains 5 - 15 % by weight of nickel and 2 - 20 % by weight of molybdenum in the form of molybdenum oxide, the percentages in each case being based on the total amount, and
b) the hydrogenation is completed in a second stage at 150 - 230° C and under 10 - 350 bar with the aid of a cobalt catalyst whose active material consists of

55 - 65 % by weight of cobalt,
15 - 20 % by weight of copper,
4 - 10 % by weight of manganese,
2 - 5 % by weight of molybdenum in the form of molybdenum oxide and
0 - 10 % by weight of other activating additives.

**Revendication**

Procédé de préparation d'alcanols en $C_3$ à $C_5$ par une hydrogénation catalysée des alcanals bruts correspondants, obtenus par l'hydroformylation d'oléfines en $C_2$ à $C_{24}$, catalysée par le cobalt, caractérisé en ce que :
a) l'hydrogénation est réalisée dans un premier stade entre 150 et 230° C sous 10 à 350 bars en présence d'un catalyseur au $SiO_2$ sur support, qui contient 5 à 15 % de son poids total de nickel et 2 à 20 % de son poids de molybdène sous forme d'oxyde de molybdène, jusqu'à une absorption d'hydrogène comprise entre 80 et 95 %, et
b) l'hydrogénation est complétée dans un deuxième stade entre 150 et 230° C sous 10 à 350 bars en présence d'un catalyseur au cobalt, dont la messe active se compose de

55 à 65 % en poids de cobalt,
15 à 20 % en poids de cuivre,
4 à 10 % en poids de manganèse,
2 à 5 % en poids de molybdène sous forme d'oxyde molybdène et
0 à 10 % en poids d'autres additifs activants.